# EUROPEAN PATENT SPECIFICATION

(11) **EP 1 819 834 B1**
(45) Date of publication and mention of the grant of the patent: **28.05.2008**
(21) Application number: 05817922.7
(22) Date of filing: 30.11.2005
(51) Int. Cl.: C12Q 1/68

(54) **TOOLS AND METHODS FOR THE QUANTIFICATION OF DNA REPEATS**
WERKZEUGE UND VERFAHREN ZUR QUANTIFIZIERUNG VON DNA-WIEDERHOLUNGSSEQUENZEN
OUTILS ET PROCEDES DE QUANTIFICATION DES UNITES DE REPETITION D'ADN

(30) Priority: 30.11.2004 GB 0426188
(43) Date of publication of application: 22.08.2007
(73) Proprietor: K.U. Leuven Research and Development, 3000 Leuven (BE)
(72) Inventor: CUPPENS, Harry, B-3000 Leuven (BE); NUYTTEN, Hilde, B-3360 Korbeek-Lo (BE); CASSIMAN, Jean-Jacques, B-3050 Oud-Heverlee (BE)
(74) Representative: Bird, Ariane
(86) International application number: PCT/BE2005/000177
(87) International publication number: WO 2006/058395

(56) References cited:
- WO-A-03/070980
- PARDIGOL ANDREAS ET AL: "A simple procedure for quantification of genetically modified organisms using hybrid amplicon standards." EUROPEAN FOOD RESEARCH AND TECHNOLOGY, vol. 216, no. 5, May 2003 (2003-05), pages 412-420, XP002374753 ISSN: 1438-2377
- TAVERNIERS ISABEL ET AL: "Cloned plasmid DNA fragments as calibrators for controlling GMOs: different real-time duplex quantitative PCR methods." ANALYTICAL AND BIOANALYTICAL CHEMISTRY, vol. 378, no. 5, March 2004 (2004-03), pages 1198-1207, XP002374754 ISSN: 1618-2642
- LALLEMAND F ET AL: "Quantitative analysis of human herpesvirus 8 viral load using a real-time PCR assay" JOURNAL OF CLINICAL MICROBIOLOGY, vol. 38, no. 4, April 2000 (2000-04), pages 1404-1408, XP002909016 ISSN: 0095-1137
- KE L D ET AL: "A reliability test of standard-based quantitative PCR: exogenous vs endogenous standards" MOLECULAR AND CELLULAR PROBES, vol. 14, no. 2, April 2000 (2000-04), pages 127-135, XP004435437 ISSN: 0890-8508

### SEQUENCE LISTING

<110> K.U.LEUVEN RESEARCH AND DEVELOPMENT
<120> TOOLS AND METHODS FOR THE QUANTIFICATION OF DNA REPEATS
<130> LRD-PCT-528
<140> not yet known
   <141> 2005-11-30
<150> GB 0426188.9
   <151> 2004-11-30
<160> 12
<170> PatentIn version 3.2
<210> 1
   <211> 20
   <212> DNA
   <213> human
<400> 1
   tccaaaggag ccagcctctc 20
<210> 2
   <211> 25
   <212> DNA
   <213> human
<400> 2
   aaaaagttca tttcacttct gcgtc 25
<210> 3
   <211> 21
   <212> DNA
   <213> human
<400> 3
   ccagccatca gccatgaggg t 21
<210> 4
   <211> 20
   <212> DNA
   <213> human
<400> 4
   tggtttacat gtcgcacgtc 20
<210> 5
   <211> 29
   <212> DNA
   <213> human
<400> 5
   taggtcgacc agccatcagc catgagggt 29
<210> 6
   <211> 26
   <212> DNA
   <213> human
<400> 6
   actcgagtgg ttacatgtcg cacgtc 26
<210> 7
   <211> 18
   <212> DNA
   <213> human
<400> 7
   ttgcgtcagc agtggagg 18
<210> 8
   <211> 29
   <212> DNA
   <213> human
<400> 8
   caatgtctct attctgcctg cccgatctt 29
<210> 9
   <211> 22
   <212> DNA
   <213> human
<400> 9
   aacaggtgcc ttgaattttg gt ₂2
<210> 10
   <211> 21
   <212> DNA
   <213> human
<400> 10
   acaaattggc acctgtggtc t 21
<210> 11
   <211> 26
   <212> DNA
   <213> human
<400> 11
   cctggaacaa aatgctgcaa aaagcc 26
<210> 12
   <211> 19
   <212> DNA
   <213> human
<400> 12
   gcagcttctt ggcctcctc 19

## Description

### FIELD OF THE INVENTION

The invention relates to an assay method allowing the quantification of the copy number of a repeated nucleic acid sequence in a genetic sample. Typically such sample comprises the genome of a microbial, plant, animal or human subject. Furthermore, the invention provides a particular example wherein the assay is used to determine the susceptibility to disease of a subject. Such information can be used in selecting the optimal treatment for a particular diseased subject.

### BACKGROUND OF THE INVENTION

Variation in the human genome is present in many forms, including single-nucleotide polymorphisms (SNP's), small insertion-deletion polymorphisms, variable numbers of repetitive sequences and genomic structural alterations. Molecular genetic and cytogenetic analyses have catalogued many variations in the human genome but little is known about large scale copy-number variations (LCVs) that involve gains or losses of several kilobases to hundreds of kilobases of genomic DNA among phenotypically normal or comparable individuals. Recently, lafrate et al. identified 255 loci across the human genome that contain genomic imbalances among unrelated individuals [1] However, it can be expected that more such loci will be identified. Many of these regions overlap with known genes and some encompassed one or more entire genes. The authors suggested that the formation of these LCVs probably reflects tandem copy number changes and they hypothesised that this heterogeneity may underlie certain human phenotypic variation and susceptibility to disease. Testing this hypothesis requires a system allowing a fast, robust and accurate estimation of the copy number of a repeated nucleic acid sequence. Only in this way, association studies can be carried out seeking a correlation between the susceptibility to a given disease and the copy number within a given repeat.

A whole array of qualitative techniques and assays is available for the detection of SNPs, small repeats (dinucleotide repeats, trinucleotide repeats, up to variable tandem nucleotide repeats) and small deletions/insertions, given their well established involvement in diseases; this in contrast to quantitative genetic DNA tests. The analysis of DNA sequence copy number is mostly used in the development, production and evaluation of the genetic stability of cell lines. Traditionally, plasmid copy numbers of cell lines are determined by quantitating band densities produced through Southern blot analysis. Quantitation of band densities from Southern blots is also used in rare X-linked diseases caused by a deletion of a gene, or gene fragment, such as in Duchemme muscular dystrophy, for the determination of the carrier status in symptomless female carriers. More recently, quantitative PCR based alternatives have become available to perform copy number analysis (for example: http://www.lark.com/services/guantitative por.html). Another technique is MAPH (multiplex amplifiable probe hybridization) which was used for the determination of DNA repeats [2], however this technique is rather difficult and too laborious.

Quantitative analysis is however most commonly used and known for the determination of transcript (RNA) levels. In most quantitative transcript assays, up to more than 10-fold differences are determined, and therefore error rates do less interfere with the result.

Quantitative DNA assays may aim at the determination of less than 1-fold differences, and error rates will therefore interfere with the result, such that more precautions will be required in quantitative DNA determination than in the current quantitative RNA determination in order to obtain correct results. In a typical quantitative experiment, standard curves are generated using control samples. The copy number of a transcript/DNA fragment is relatively determined against another reference transcript/DNA fragment, this for different control samples. In general, a control sample is prepared in which a fixed amount of a reference fragment is mixed with a known amount of the fragment under investigation. Typically, different control samples are used, each of them having a different ratio of the concentration of the fragment under investigation to the concentration of the reference fragment, which is usually the same in all control samples. Using these control samples a standard curve is determined from which the concentration/copy number can be deduced of the fragment under investigation in the studied samples. It is obvious that this method is prawn to pipetting errors, which can not be tolerated when small quantitative differences need to be determined. So, although the latter methods allow a fast and quantitative estimation of the DNA copy number they are prawn to such errors that they are not suitable to accurately determine the DNA copy numbers of a given sequence in a large number of samples.

PARDIGOL et al. (European Food Research and Technology, vol. 216, no. 5, May 2003, pages 412-420) discloses hybrid amplicon quantification standards for the quantification of transgene sequences, each standard comprising a single copy of a reference gene (HMG or lectin) and a transgene sequence (p35S or RRS) (p. 413, right-hand column; p. 414, right-hand column).

The present invention provides control constructs and methods for the determination of the copy number of nucleotide sequence repeats, which are not affected by pipetting and dilution errors, allowing therefore the accurate determination of the number of repeats of the fragment under investigation. The method depends on the use of said control constructs, which can be used in many different quantitative assay systems and more particularly quantitative PCR assays. The sensitivity and accuracy of the methods of the present invention are such that they allow the detection of 1 repeat difference between 2 samples with a limited number of measurements per sample. They allowed to demonstrate for the first time that variations in copy-numbers of the genes encoding human beta-defensins 2-6 can be correlated to the severity of disease in an individual. More specifically, the assay method of the present invention allowed to correlate the severity of lung disease in cystic fybrosis (CF) patients with the number of repeats of the DNA sequences encoding beta-defensins 2 to 6. Also in the development of chronic obstructive pulmonary disorder the copy number of these genes seems to be a predisposing factor. Further, the distribution of the copy number of the genes encoding said defensins appeared to differ between populations having a different age range, suggesting that certain copy numbers are associated with early mortality. Defensins are small cationic antimicrobial and chemotactic proteins that are expressed in epithelial tissues, such as the lung epithelium. Six β-defensins are expressed in the human lung: hBD1, -2, -3, -4, -5 en -6 [3]. They are also respectively known as DEFB1, DEFB4, DEFB103A, DEFB104, DEFB105, DEFB106). Only hBD1, -2, -3 and 4 have been studied in more detail. hBD2, -3 and 4 have an inducible expression pattern, hBD1 is constitutively expressed. They all possess antimicrobial activity against a broad spectrum of bacteria, fungi and enveloped viruses. In addition, hBD1, -2 and -3 possess chemotactic activity for immature dendritic cells and memory T-cells while hBD3 and -4 elicit monocyt chemotaxis [4]. The hBD2, -3, -4, -5 and -6 genes are part of a 250 kb repeat region on chromosome 8p23 which is repeated 3-12 times per diploid genome [2], and therefore 3-12 gene products are found for hBD2, -3, -4, -5 and -6 in a diploid cell. For hBD2 it has been shown that the amount of hBD2 transcripts correlate with the number of repeats.

Cystic fibrosis (CF) is the most common lethal autosomal recessive disorder in the Caucasian population. CF is caused by mutations in the cystic fibrosis transmembrane conductance regulator channel (CFTR) gene, which is located on chromosome 7q31.3. This gene encodes a protein that is expressed in the apical membrane of exocrine epithelium cells. CFTR functions as a cAMP-induced chloride channel. CF is characterised by progressive bronchietatic lung disease, pancreatic exocrine insufficiency, chronic sinusitis and male infertility. Lung disease is the main cause of death.

More then 1000 different disease-causing mutations have been identified in the CFTR gene. F508del is the most common mutation. A broad spectrum of disease severity is observed between patients, even among patients with the same CFTR genotype, such as F508del/F508del, or even CF sibs. The CF phenotype is therefore modulated by other genetic factors, as well as environmental factors. Mannose-binding lectin, tumor necrosis factor alpha and transforming growth factor beta1 have been reported as possible modifier genes [5]. Since human β-defensins form part of our first line of defense of the lung, they are candidate modifier genes of cystic fibrosis lung disease.

COPD is a disease state characterized by airflow limitation that is not fully reversible, which is progressive and associated with an abnormal inflammatory response of the lungs to noxious particles and gasses. Chronic bronchitis and emphysema are the most common underlying disorders. Emphysema is a condition of the lung characterized by abnormal, permanent enlargement of airspace distal to the terminal bronchioli accompanied by destruction of their walls and without obvious fibrosis. Smoking is a risk factor, but since not all smokers develop COPD, other factors are involved. Defensins are possible genetic factors involved in these diseases.

### DEFINITIONS

"Variable copy-number sequence" means a nucleic acid sequence, which is repeated within the genetic material of a living organism. Said nucleic acid sequence can be a non-coding sequence or it can comprise one or more genes. Typically, the copy number of this repeated nucleic acid sequence varies among individuals of the same species. It is known that some sequence variation may exist between the copies of a repeated sequence, nevertheless the homology between two copies of a variable copy-number sequence should be more than 70%, more preferably more than 80% and most preferably more than 90%, for instance 95% or more.

### SUMMARY OF THE INVENTION

The present invention provides an assay kit comprising particular control constructs and methods for using said control constructs in the estimation of the copy number of variable copy-number sequences. Said methods are not affected by pipetting and dilution errors, allowing therefore the accurate determination of the copy number of the fragment under investigation. Using said methods it could be demonstrated that the copy number of the genes encoding human beta-defensins 2 to 6 could be used to predict an individual's predisposition to develop given diseases.

### DESCRIPTION

### Legends to the figures

- Figure 1:: Ligation of the cDNA of hBD2 in the pUC-18-hBD1 vector
- Figure 2:: TaqMan experiment with the control constructs: Linear regression analysis
- Figure 3:: Overall distribution of FEV1% value according to the amount of defensin genes (10 repeats or more are grouped together)
- Figure 4:: Amount of defensin repeats versus least square means of the according FEV1% value with standard error.
- Figure 5:: Amount of defensin repeats versus the mean FEV1% values, linear regression analyses of the means with standard error.
- Figure 6:: Relation between the copy number of the gene encoding hBD2 and the basal expression levels of hBD2
- Figure 7:: Induction of beta-defensin 2 expression by TNF-alpha
- Figure 8:: Variation of the copy number of the genes encoding hBD2-6 in populations having a different age range

### Detailed description

The invention relates to an assay method allowing the quantification of the copy number of a variable copy-number sequence within a genetic sample. The method of the invention has the advantage that it minimises the measurement errors due to concentration determination errors and/or pipetting errors by providing a set of at least two control constructs. Each control construct being an isolated nucleic acid sequence comprising the same number of copies, for instance a single copy, of a reference sequence and a different but known number of copies of a repeated nucleotide sequence. Preferably, the reference sequence and the repeated sequence are arranged concatemerically within these control constructs. Said set of control constructs is used to obtain a standard curve for the calculation of the copy number of a variable copy-number sequence in a genetic sample using a quantitative assay. Within said quantitative assay values reflecting the concentration of the repeated sequence and the reference sequence, respectively, are determined for each control construct and subsequently the value obtained for the reference sequence is used to normalise that of the repeated sequence. Said normalised values obtained for the respective control constructs allow to calculate a standard curve indicating the relation between the normalised values and the copy number of the repeated nucleotide sequence. Using this standard curve the number of repeats of a variable copy-number sequence in a genetic sample can be extrapolated after determining a value reflecting the concentration of said variable copy-number sequence and normalising said value against a value obtained for a reference sequence also comprised in said genetic sample. Preferably, the copy number of the selected reference sequence does not vary between the genetic samples. It is also preferred that the reference sequence is located close to the variable copy-number sequence within the genome comprised in the genetic sample. Therefore, in a first object the present invention provides a set of control constructs each comprising a fixed copy number of a reference sequence and a known but variable number of copies of a repeated nucleotide sequence. Preferably the copy number of the repeated sequence in said control constructs varies between 1 and 20, more preferably between 1 and 15, for instance between 1 and 10.

The invention further provides methods for using said control constructs for the quantification of the copy number of a variable copy-number sequence within a genetic sample.

In a first embodiment the reference sequence comprised in each control construct corresponds to the reference sequence used to normalise the values reflecting the concentration of the variable copy-number sequence in the genetic samples. Each control constructs further comprises a different but known number of copies of a repeated nucleotide sequence, which corresponds to at least a part of the variable copy-number sequence of which the copy number needs to be determined in a genetic sample. Preferably, the repeated sequence comprised in the control constructs corresponds to the part of the variable copy-number sequence in the genetic samples that is amplified by Polymerase Chain Reaction (PCR) in the quantitative assays described below. These control constructs can be used in different types of quantitative PCR assays. For instance when using said control constructs in a real-time TaqMan^{™} PCR the copy number of a variable copy-number sequence in a genetic sample can be determined as follows:
(i) amplifying the reference sequence and the repeated sequence of each of the control constructs using primers, which are sequence specific for the reference sequence and the repeated sequence, respectively, said amplification being preferably performed in a multiplex configuration
(ii) determining the cycle threshold (Ct-value) of the reference and the repeated sequence, respectively, for each control construct
(iii) calculating the difference between the Ct-value of the reference sequence and the Ct-value obtained for the repeated sequence (Ct-ref - Ct-repeated) for each control construct
(iv) determining a relationship between the number of copies of the repeated sequence, and the difference between Ct-ref - Ct-repeated.
(v) using this relationship to calculate the copy number of the variable copy-number sequence in a genetic sample comprising an unknown copy number after determination of the Ct-ref - Ct-variable for that sample, preferably using the same primers as used in step (i).

An alternative method for the estimation of the copy number of a variable copy-number sequence in genetic samples comprises PCR amplification of the reference and the repeated sequence followed by the quantification of the obtained PCR products. In a particular embodiment sequence specific primers are used whereby one of the primers for the reference sequence and one of the primers for the repeated sequence comprises a tag, for instance a biotinyl molecule, allowing the purification of the PCR products from unreacted primers. The other primer for the reference sequence and the other primer for the repeated sequence comprises a label, for instance a fluorescent label, allowing the detection of the PCR-product in function of its concentration. Preferably said primer for the reference product comprises a different label, for instance a different fluorochrome, than said primer for the repeated sequence. A PCR is performed with each control construct using said primers, preferably in a multiplex configuration, and after isolation of the obtained PCR-products the signal associated with the PCR-product of the repeated sequence is detected and normalised against the signal obtained for the PCR-product of the reference signal. Said normalised values allow calculating a standard curve indicating the relation between the normalised values and the copy number of the repeated nucleotide sequence. Using this standard curve the number of repeats of a variable copy-number sequence in a genetic sample can be extrapolated after PCR amplification using said primers and detection of the obtained PCR product of respectively the reference sequence and of the part of the variable copy-number sequence. A variant of this method, which does not require labelling of the primers, comprises the PCR amplification, preferably in a multiplex configuration, of the reference and repeated sequence of the different control constructs. Thereafter, the PCR product of each sample is bound to a membrane (dot-blotting) or beads and the bound material is hybridised with sequences specific to the reference sequence and the repeated sequence, respectively. Alternatively, said sequences specific to the reference sequence and the repeated sequence, respectively, are, bound to a membrane (reversed-dot-btot) or beads and the PCR products are hybridised to the bound sequences. The strength of the hybridisation signal of the repeated sequence is normalised against the hybridisation signal of the reference sequence for each of the control constructs. Said normalised values are then used to set the standard curve. Using this standard curve the number of repeats of a variable copy-number sequence in a genetic sample can be estimated.

In a second embodiment the reference sequence comprised in each control construct does not correspond to the reference sequence used to normalise the values reflecting the concentration of the variable copy-number sequence in the genetic samples. Each control construct further comprises a different but known number of copies of a repeated nucleotide sequence, which differs from the variable copy-number sequence of which the copy number is to be determined in the genetic sample. To estimate the copy number of a variable copy-number sequence, a reference sequence and a part of the variable copy-number sequence are amplified using sequence specific primers. At least one primer of the reference sequence comprises a 5' adapter sequence, said sequence corresponding to at least a part of the reference sequence comprised in the control constructs. In addition, at least one primer of the variable copy-number sequence comprises a 5' adapter sequence, said sequence corresponding to at least a part of the repeated sequence comprised in the control constructs. After the amplification of the reference and part of the variable copy-number sequence using the said primers, the isolated PCR products are hybridised with sequences complementary to the respective adapters. The respective hybridisation signals are detected and the signal measured for the amplified variable copy-number sequence is normalised against the hybridisation signal obtained for the reference sequence. This normalised hybridisation signal allows the extrapolation of the copy number of the variable copy number in the genetic sample after setting a standard curve using solutions of the respective control constructs. A sample of each control construct is hybridised with the sequences complementary to the respective adapters. It is clear that the hybridisation signal obtained with the sequence complementary to the adapter of the primer of the reference sequence reflects the concentration of the reference sequence in the control construct sample. On the other hand, the hybridisation signal obtained with the sequence complementary to the adapter of the primer of the reference sequence reflects the concentration of the repeated sequence in the control construct. After normalisation of the hybridisation signal obtained for the repeated sequence against that obtained for reference sequence for each control construct, a standard curve can be calculated indicating the relation between the normalised hybridisation values and the copy number of the repeated sequence. Using this standard curve and the normalised hybridisation signal obtained for a given genetic sample the copy number of the variable copy-number sequence in said sample can be extrapolated. An important advantage of the control constructs of the second embodiment is that they allow the quantification of the copy number of the different variable copy-number sequences, using the same control constructs. Only the primers need to be customised in function of the variable copy-number sequence of interest and the selected reference sequence. However, the availability of different sets of control constructs, each set comprising a different reference sequence and repeated sequence, makes it possible to simultaneously quantify the copy number of different variable copy-number sequences in a single assay, for instance a multiplex quantitative PCR assay.

A third embodiment of the present invention provides control constructs according to the first or second embodiment comprising multiple reference sequences and multiple repeated sequences. Such control constructs can be used to simultaneously quantify the copy number of different variable copy-number sequences in a single assay, for instance a multiplex quantitative PCR assay.

The person skilled in the art will understand that the control constructs and methods of the present invention allows to develop assay systems for all variable copy-number sequences (more than 255 loci) in the human genome which are known to comprise sequences of which the copy number varies among individuals. In a preferred embodiment these variable copy-number sequences are larger than 2kb and more preferably larger than 5 kb. The availability of these assays certainly will allow the development and implementation of genetic tests evaluating the relationship between the severity of and/or predisposition to a disease of a subject and the copy number of a given sequence in its genome. This is exemplified by the finding that the method of the present invention allowed to correlate the severity of lung disease in cystic fybrosis (CF) patients that are homozygous for the F508del mutation with the copy number of the variable copy-number sequence comprising the genes encoding beta-defensins 2 to 6. Furthermore, it was observed that the distribution of the said beta-defensin repeats in a population of COPD patients and more particularly, emphysema patients, differed from the distribution thereof in a control population. These findings are of particular relevance knowing that the beta-defensins are important antimicrobial and chemotactic proteins in the lungs and as such important factors in the early response against pathogenic infection. Therefore, in a second object the invention provides a method for predicting the severity of lung disease of a patient and/or predisposition of a subject to develop lung disease based on the determination of the copy number of the variable copy-number sequence comprising the genes encoding beta-defensins 2 to 6. In a more preferred embodiment, this method is used to predict the lung disease severity in CF patients. It is clear that the prediction of the potential severity of a disease in a particular patient assists the clinician in deciding on providing a preventive and/or more intensive treatment to such patient.

The results of this study clearly shows that from a copy number from 3 to 6 of the variable copy-number sequence comprising the genes encoding beta-defensins 2 to 6, the copy number correlated positively with the severity of the disease. Furthermore, it has been shown for beta-defensin 2 (hBD2) that the amount of hBD2 transcripts correlates with the copy number, indicating that the expression of this protein is at least in part determined by the copy number of the hBD2 gene. These findings suggest that in patients having a low copy number of the variable copy-number sequence comprising the genes encoding beta-defensins 2 to 6, the severity of the disease is related to low levels of one or more of these defensins. Therefore, in a third object the present invention relates to the use of a beta-defensin or a derivative thereof and more particularly any of the beta-defensins 2 to 6 or derivatives thereof for the preparation of a medicin. In a particular embodiment this medicin is used to treat CF patients having a low copy number of the variable copy-number sequence comprising the genes encoding beta-defensins 2 to 6.

Further, the distribution of the copy number of the genes encoding said defensins appeared to differ between populations having a different age range, suggesting that certain copy numbers are associated with early mortality. This finding suggest that certain copy numbers of said genes are associated with one or more life threatening conditions.

### Example 1: Development of an assay system for the quantification of the copy number of the genes encoding hBD2-6 and its use in the study of the genetic basis of cystic fibrosis.

### Studied populations

For the cystic fibrosis association studies, 47 Belgian, 37 South-Italian and 52 Czech CF patients were investigated. Only CF patients homozygous for the F508del mutation were included, in order to minimise the effect of variability of the CFTR genotype on the CF phenotype. The FEV1 values, which are a measure of lung function, were taken from the clinical records of the patients. Only the disease status at a small age range was studied, i.e. the age of the patients varied between 11 and 15 years, in order to normalise for age. The FEV1 values were normalised to FEV1% according to Knudson et al. [6].

### Preparation of the control constructs

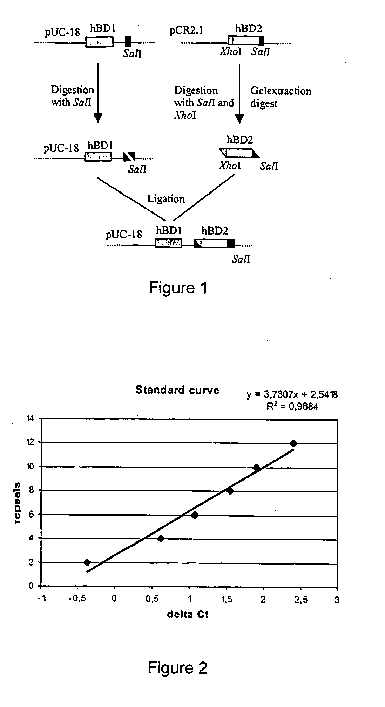

In a molecular biological assay, the copy number of a gene that is part of repeat should be determined relatively to a gene that is not part of that repeat. Since that hBD1 is not part of the repeat region and located next to the repeat region containing the hBD2-6 genes, hBD1 could be used for normalisation of a gene that is part of the repeat such as hBD2. In fact, the reference DNA fragment should preferably be a DNA fragment next to the repeat DNA fragment, which is more likely to have a similar local DNA structure and accessibility. Preferably for each repeat number, a control construct is needed containing the respective number of the repeated hBD2 fragments and 1 hBD1 fragment. Therefore, for each repeat number, we decided to construct a concatemeric vector, i.e. a vector containing one hBD1 fragment and the respective number of hBD2 fragments, also in a concatemeric fashion. They were prepared in vectors, allowing easy generation of these control materials. In first instance, pUC18-hBD1 and pUC-18 hBD2 were generated. For this purpose, RNA was extracted from nasal epithelial cells and transformed into cDNA. PCR was then performed with the hBD1 primers: 5'-TCCAAAGGAGCCAGCCTCTC-3' (SEQ ID No 1) and 5'AAAAAGTTCATTTCACTTCTGCGT C-3' (SEQ ID No 2) and hDD2 primers: 5'-CCAGCCATCAGCCATGAGGGT-3 (SEQ ID No 3) and 5'-TGGTTTACATGTCGCACGTC-3' (SEQ ID No 4). The PCR-product was ligated into the Sma/BAP site of pUC-18 and sequencing was performed to test if the cloning was successfull. In order to ligate an hBD2 fragment into the pUC18 *Sal*I restriction site of pUC18-hBD1, a newhBD2 vector was made. PCR was performed with adaptor hBD2-primers containing restriction sites at their 5' end: hBD2XalI: TAGGTCGACCAGCCATCAGCCATGAGGGT-3' (SEQ ID No 5) and hBD2*Xho*I: 5'-ACTCGAGTGGTTACATGTCGCACGTC-3' (SEQ ID No 6). The hBD2 fragment was then cloned in PCR2.1 (Invitrogen), using the *Sal*I and *Xho*I restriction sites. The PCR2.1-hBD2 vector was then digested with *Sal*I and *Xho*I*.* The insert was separated from the vector by gel electrophoreses on a 2% agarose gel and purified using the Qiagen gel extraction kit. The hBD2 insert was ligated in the defosforylated pUC-18-hBD1 vector and cloned in XL1blue electrocompetent cells. The insertion was controlled by PCR and sequencing. The plasmid DNA was obtained by extraction with nucleobond AX500. The subsequent constructs where made by digesting the pUC-18hBD1-hBD2 vector with *Sal*I and ligating the hBD2-insert in the defosforylated vector (Figure 1). Indeed, after ligation of an hBD2 fragment in pUC18, only the last Sall recognition site remained intact, such that this vector could be used for an additional ligation of an hBD2 fragment in the *Sal*I site. This latter step was repeated for each construct until the appropriate number of hBD2 repeats was obtained.

### Real time PCR

DNA was isolated from whole blood samples using standard molecular biology procedures. For real time PCR, 100 ng of genomic DNA was used (patient samples) and 100 ng of plasmid DNA (control constructs).

The diploid copy number of hBD2 of each sample was determined by real time PCR with the qPCR Core kit (Eurogentec) on an ABI 7000 machine (Applied Biosystems). For each sample, a PCR was performed with the following primers and probe for hBD1:
5'-TTGCGTCAGCAGTGGAGG-3' (SEQ ID No 7),
5'-VIC-CAATGTCTCTATTCTGCCTGCCCGATCTT-TAMRA-3' (SEQ ID No 8)
and 5'-AACAGGTGCCTTGAATTTTGGT-3' (SEQ ID No 9)
and for hBD2:
5'-ACAAATTGGCACCTGTGGTCT-3' (SEQ ID No 10),
5'-FAM-CCTGGAACAAAATGCTGCAAAAAGCC-TAMRA-3' (SEQ ID No 11)
and 5'-GCAGCTTCTTGGCCTCCT C-3' (SEQ ID No 12).

The PCR reaction was performed in a 96 well clear optical reaction plates (Applied Biosystems); for each sample, 3 independent PCR reactions were performed and each of these 3 assays was even performed in duplo. For each real-time PCR reaction, 25-µl solutions containing 2 µl of cDNA, 1x qPCR Master Mix (Eurogentec), at 200 nM of each sense and antisense primer, and 250 nM TaqMan probe. The reaction conditions were set at 50°C for 2 min and 95°C for 10 min, followed by 40 cycles of 95°C for 15 s and 60°C for 1 min.

Each 96 well plate contained 6 hBD2 control constructs. The delta Ct value of the control constructs (Ct hBD1 - Ct hBD2) was correlated to the amount of hBD2 in each plasmid. In this way a standard curve could be constructed for each 96-well plate; which could then be used to determine the exact hBD2 copy number of the different samples under investigation.

### Statistical analyses

Statistical analyses was performed using the SYSTAT package, release 7.0 (SPSS Inc. Chicago, IL, USA). Statistical tests were considered significant when their type I error was less than 0.05.

### Results

### Control constructs

Six concatemeric control constructs were generated in pUC-18 containing one 1 hBD1 fragment and 1 up to 6 hBD2 fragments. TaqMan PCR with these control constructs showed a linear relationship between the amount of hBD2 and the delta Ct-value (Ct_{hBD1}-Ct_{hBD2}) (Figure 2). Therefore a standard curve from these constructs can be made and used to interfere from the delta-Ct values of the samples under investigation the diploid copy number of hBD2 repeats in these samples.

### Analyses of individuals

The diploid hBD2 copy number was determined in 135 Belgian, Czech and South-Italian CF patients homozygous for the F508del mutation. All TaqMan experiments were independently performed 3 times. On each 96-well plate, all tests were performed in duplo and contained all control constructs, so that a standard curve was made for each experiment/plate. Over the three experiments the standard deviation of the amount of repeats was less then 1.

### Cystic fibrosis association study

For the CF-study, only patients aged between 11 and 15 years were taken up in the study. It was then tested if the hBD2 copy number correlates with lung disease severity in these patients. As a parameter for cystic fibrosis lung disease severity, FEV1% values corrected according to Knudson et al. [6] was taken.

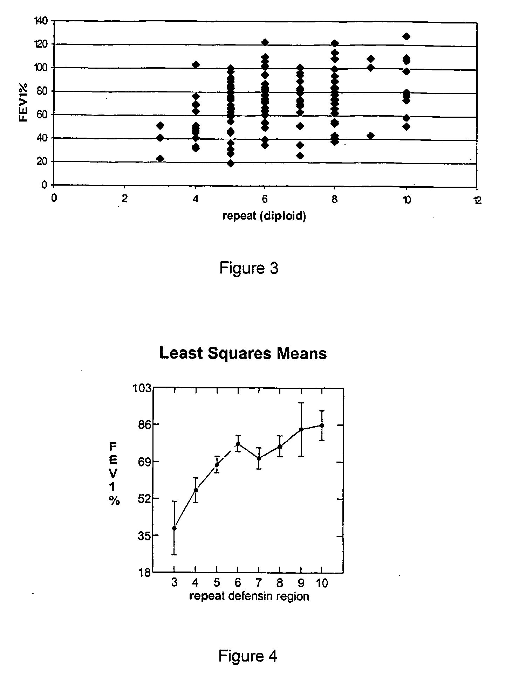

In Table 1, an overview of the studied population is shown in which the mean FEV1% value is correlated to the mean repeat value of the total patients in each population. In the Italian population, significant more repeats were found compared to the Belgian and Czech populations (ANOVA: p = 0.00019). The FEV1% values were not significantly different in the three populations (p = 0.8). In Figure 3 the amount of repeats are plotted against the FEV1% values. Patients with 10 or more repeat were grouped together, because the numbers were very small and 12 or more repeats are out of the range of the standard curve. The clouds of dots indicate that there is a positive correlation between the FEV1% values and the amount of repeats.

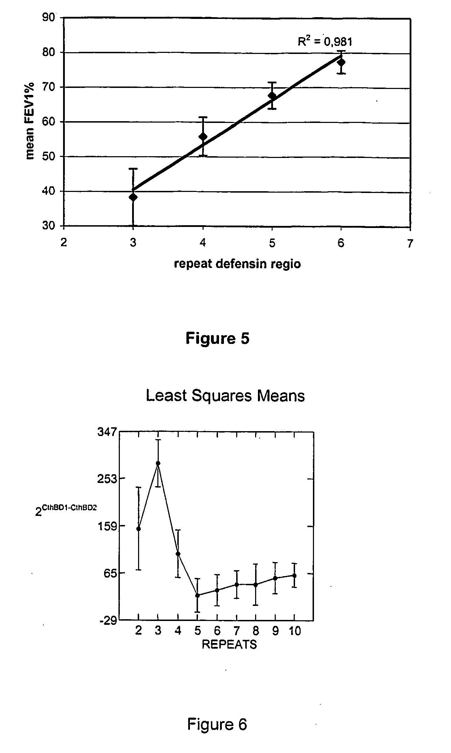

In Table 2 an overview is given for the different repeats and the mean FEV1% value for each repeat. The table shows that a higher amount of repeats correlates with a higher FEV1% value. The average repeat number found in the whole population is 6. The results listed in Table 2 are plotted in Figure 4, and for each repeat the standard error is indicated. From 3 to 6 repeats the amount of repeats correlates strongly positively with the FEV1% values. A more detailed look of the correlation between the amount of repeats from 3 to 6 repeats and the FEV1% shows that this correlation seems to be linear in this range as shown in Figure 5 with a R²-value of 0.981. Moreover, the 0.95 confidence intervals do not overlap. In the smaller repeat range, one repeat unit more or less has already a significant effect on the FEV1% value.

An ANOVA test on the whole dataset with the FEV1% as independent variable and the amount of repeats as factor effect (with 10 or more repeats grouped together) gives a p-value of 0.0015. When we divide the data set into three groups as listed in Table 3, the ANOVA test becomes even more significant with a p-value of 0.0002.

When each population is studied separately, the correlation between FEV1% values is significant within each population (Table 4). We have divided each data set into two groups and performed a student t-test. The South-Italian population is divided differently, because the distribution of the repeats was different compared to the other populations studied, i.e. a higher proportion of repeats is found in that population. An ANOVA test on the Czech population only gave a p-value of 0.038.

### EXAMPLE 2: Effect of the copy number of the genes encoding hBD2-6 in a study on the genetic basis of COPD

### Studied populations

For the COPD association study, 69 healthy Belgian smoking control samples and 44 emphysema patients were compared.

### Results

### Analyses of individuals

The diploid hBD2 copy number was determined in 69 Belgian smoking control patients and 44 Belgian COPD patients. All TaqMan experiments were independently performed 3 times. On each 96-well plate, all tests were performed in duplo and contained all control constructs, so that a standard curve was made for each experiment/plate. Over the three experiments the standard deviation of the amount of repeats was less then 1.

### COPD association study

The study showed that emphysema patients had more repeats than control individuals (P-value = 0.006)

### Example 3: Correlation of hBD2 transcript levels with the number of hBD2 repeats

### Tissue culture of nasal epithelial cells

Nasal polyps were obtained from about 50 patients having rhinosinusitus, in whom polyps were removed for medical reasons by a surgical intervention. From these tissue samples, monolayers of epithelial cells were grown as described before [7].

Immediately after surgical isolation, the tissue samples were placed in Dulbecco's Modified Eagles Medium (DMEM) (Invitrogen), supplemented with 100 U of penicillin (PE) (Invitrogen) and 100 mg/ml streptomycin (ST) (Invitrogen), before transportation to the laboratory. After washing the tissue samples 3 times in a sterile saline solution (0.9% NaCl) containing PEST, the samples were cut into small pieces (using a sterile surgical blade) and placed on a tumble mill in a container with a solution of pronase (1 mg/ml) (Sigma) for 16-24 h in a cold chamber (4°C) to enable enzymatic dissociation of the epithelium. After gentle shaking, the remaining large pieces of stroma in the solution were removed and stored at -70°C. DNA was extracted out off the remaining stroma with a chloroform isoamylalcohol extraction (Invitrogen). The dissociated epithelial cells were washed 3 times with DMEM plus PEST by centrifugation (700 g); the first time with 10% foetal calf serum to inactivate the enzyme. The retrieved pellet of cells was suspended in DMEM plus PEST and plated in T-25 Falcon tissue culture flasks. The cell cultures were incubated at 37°C for 1 hour, where the nasal epithelial cells remain in suspension, while the fibroblasts attach to the cell culture flask. After one hour, the suspensions were isolated and the cells were counted using a coulter counter and seeded in collagen coated T-75 tissue culture flaks with DMEM plus PEST plus Ultroser G containing 10 000 cells/cm2. The medium was refreshed after the first day and subsequently every 2 days until confluent monolayers were observed via inverted phase contrast microscopy. After confluence, the cells where harvested: collagenase was added to dissolve the collagen, the cells were washed 2 times with DMEM plus PEST by centrifugation (700g). The cells were split in two groups. The first group was used for RNA extraction, the pellet was resuspended in PBS and washed 2 times with PBS by centrifugation (700g). After removing the PBS, the pellet was stored at -70°C for later RNA extraction. RNA was isolated from the frozen cells with Trizol reagent (Invitrogen).

The second part of the cells was resuspended in 10 ml Versene (Invitrogen) and after centrifugation resuspended in 1.5 ml Trypsine (Invitrogen). After 7 minutes, the cells were washed in 10 ml DMEM plus PEST plus 10% FCS. After centrifugation (700g), the pellet was resuspended in 3 ml 10% GCS in DMEM F12 plus PEST plus 5% DMSO and frozen in liquid nitrogen for later use, such as re-culturing.

### Air liquid interface culture

Primary nasal epithelial cell cultures having 4 hBD2 repeats and 8 hBD2 repeats were re-cultured, starting from the frozen liquid nitrogen stock. The cells were unfrozen and cultured in a collagen coated T75 culture flask. After confluence, the cells were transferred to a 6 well plate containing transwell inserts coated with collagen. The membrane was wetted with medium for 10 min and then the medium was removed. The lower reservoir was first filled with 1 ml culture medium. The upper reservoir was filled with culture medium containing approximately 1*10⁶ cells. After confluence, the upper medium was removed and cells were kept in air liquid interface. Differentiation was checked by microscopy (cilia beating).

### hBD2 transcript level analysis in the primary nasal epithelial cell lines.

RNA (1µg) was reverse transcribed into cDNA using superscript reverse transcriptase. For each sample, 3 independent real time PCR assays with the HBD1 and hBD2 primers [SEQ ID No 7-8-9-10-11-12] using cDNA as template, were performed, and each of these 3 assays was performed in duplo. A delta Ct value of n equals a copy number variation of 2ⁿ. The variation in expression was then calculated by 2^{CthBD1-CthBD2} and evaluated against the number of the repeats found at the genomic level.

### Stimulation with TNF-alpha

Tumor necrosis factor alpha (TNFα) is known to increase hBD2 transcription in epithelial cells[8]. TNFα (R&D Systems) was dissolved in phosphate buffered saline (PBS) + 0.1% bovine serum albumin (BSA) at a concentration of 10 µg/ml.

This TNFα stock solution was 100x diluted in DMEM-F12 plus PEST. The upper layer of the cells was washed with PBS before applying 100 µl TNF-alpha solution to the cells. The 12-well plates were incubated in a 5% CO₂ incubator for different time periods. After incubation, the epithelial cells were washed 3 times with PBS and the RNA was extracted from the cells with an RNA extraction kit (Qiagen). DNA was removed during RNA extraction with a RNAse-free DNAse kit (Qiagen). The RNA was stored at -70°C.

RNA (1µg) was reverse transcribed into cDNA using superscript reverse transcriptase. The relative amount of IL-8 and hBD2 was determined with real time PCR on the ABI 7000. IL-8 was used as a control. For each cDNA sample: IL-8, hBD2, and the housekeeping gene human porphobilinogen deaminase (hPBGD) were analysed. The obtained Ct values, i.e. the number of PCR amplification rounds required to reach a certain threshold amount of amplification products in the exponential phase of the PCR reaction, is directly correlated to the amount of target mRNA. The higher the amount of target mRNA (transcribed into cDNA) in the sample, the lower the Ct value. To correct for the total amount of cDNA present in the sample, the housekeeping gene (hPBGD) was amplified in the same assay and its Ct value was subtracted from the Ct value of the gene under analysis. This subtraction gives the ΔCt value, which represents the relative amount of transcripts of the gene under analysis in the sample. A change in ΔCt = n represents a change in expression of the gene under study with a factor 2ⁿ. In order to compare different experiments, a negative control was included in each experiment.

### Number of repeats versus expression on RNA level

Since a higher DNA repeat number correlates with a higher gene dosage of the different genes located in the DNA repeat, it is expected that a higher number of transcripts and proteins will be derived from these genes when a higher repeat number is found. We therefore tested the correlation between the number of DNA repeats and hBD2 transcript levels in 50 primary nasal epithelial cell cultures. Nasal epithelial cells are very prone to environmental factors, such as infections, affecting the transcriptome. By culturing biopsies of nasal epithelial cells in well-controlled conditions for 6 weeks, these environmental factors will be neutralised allowing more accurate genome-transcriptome correlations. First of all, the number of repeats in these 50 different samples was determined at the genomic level. Subsequently the expression of hBD2 was evaluated. We decided to determine the level of hBD2 transcripts, because the basal level of the other transcripts is below the detection limit [8, 9]. A positive correlation was found between the number of defensin repeats and hBD2 transcript levels (ANOVA P-value = 0.004), and therefore a higher copy-number of defensin repeats equals with a higher basal expression of hBD2. Moreover a plateau effect was also observed. From 2-6 repeats, there was a strong increase in hBD2 expression (ANOVA P-value = 0.01), in patients with more than 6 repeats the number of repeats did not have an effect on the basal expression level (ANOVA P-value = 0.94, Figure 6).

The same plateau effect is observed in the CF association study, where a strong modulating effect is seen from 3 to 6 repeats and only a mild effect in patients with more than 6 repeats.

### Induction of beta-defensin2 expression by TNF-alpha

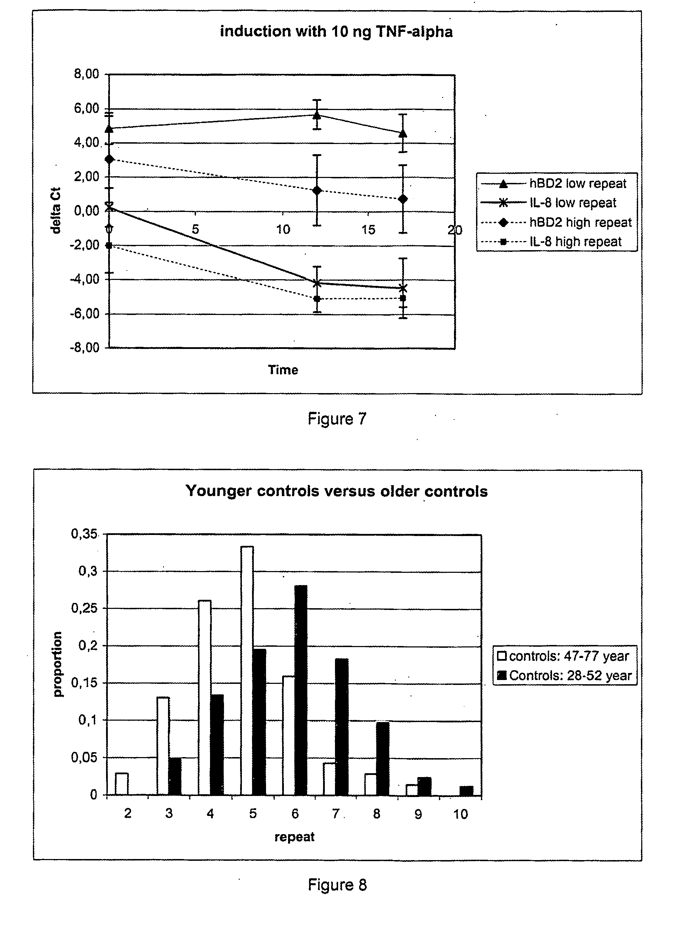

We evaluated the expression of hBD2 and IL-8 after induction with 10 ng TNF-alpha. It was previously shown that after 12 hours of induction, the expression of hBD2 and IL-8 is significantly induced in epithelial tissue [8]. We have chosen hBD2, because we observed a correlation between the number of repeats and the basal expression of this defensin. We also included IL-8 in the study as a control for the experiment. TNF-alpha induces IL-8 expression, but it is expected that IL-8 expression is not altered by the number of defensin repeats. We selected two cell lines with 4 repeats and 1 cell line with 8 repeats. After 17 hours of induction, hBD2 expression was not induced by TNF-alpha (P-value: = 0.899, Figure 7) in the cell lines with 4 repeats, while the cell line with 8 repeats showed a significant induction by TNF-alpha (P-value = 0.05). The induction of IL-8 was similar in the cell lines having either a low or high number of defensin repeats (P-values; 0.00 and 0.045)

### Example 4: Variation of the copy number of the genes encoding hBD2-6 in populations having a different age range

The number of defensin repeats was determined in two control groups that varied in age range. A different distribution of repeats was found in the two controls groups, i.e. in the older control group, a lower proportion of individuals carried a high defensin repeat number. A possible explanation is that a high repeat number of this defensin region is deleterious in common complex diseases, e.g. lung diseases, auto-immune diseases, amongst others, and these individuals are lost because they die earlier and therefore are underrepresented in the older group of individuals (Figure 8). This is an indication that defensins may be involved in other diseases than in CF, and not necessarily in the same way as in a pediatric disease such as CF.

### References

[1] lafrate, A.J., Feuk, L., Rivera, M.N., Listewnik, M.L., Donahoe, P.K., Qi, Y., Scherer, S.W. and Lee, C. (2004) Detection of large-scale variation in the human genome. Nat Genet.
[2] Hollox, E.J., Armour, J.A. and Barber, J.C. (2003) Extensive normal copy number variation of a beta-defensin antimicrobial-gene cluster. Am J Hum Genet 73, 591-600.
[3] Schutte, B.C. and McCray, P.B., Jr. (2002) [beta]-defensins in lung host defense. Annu Rev Physiol 64, 709-748.
[4] Yang, D. et al. (1999) Beta-defensins: linking innate and adaptive immunity through dendritic and T cell CCR6. Science 286, 525-528.
[5] Accurso, F.J. and Sontag, M.K. (2003) Seeking modifier genes in cystic fibrosis. Am J Respir Crit Care Med 167, 289-290.
[6] Knudson, R.J., Lebowitz, M.D., Holberg, C.J. and Burrows, B. (1983) Changes in the normal maximal expiratory flow-volume curve with growth and aging. Am Rev Respir Dis 127, 725-734.
[7] Jorissen, M., et al., The preservation and regeneration of cilia on human nasal epithelial cells cultured in vitro. Arch Otorhinolaryngol, 1989. 246(5): p. 308-14.
[8] Vankeerberghen, A., et al., Differential induction of human beta-defensin expression by periodontal commensals and pathogens in periodontal pocket epithelial cells. J Periodontol, 2005. 76(8): p. 1293-303.
[9] Claeys, S., et al., Human beta-defensins and toll-like receptors in the upper airway. Allergy, 2003. 58(8): p. 748-53.

### Tables

**Table 1: F508de1/F508de1 CF population: Origin of the patients and the corresponding mean FEV1% value and mean amount of repeats.**

| **Population** | **Individuals** | **Mean FEV1%** | **Mean repeat value** |
|---|---|---|---|
| Belgian | 46 | 73 | 6 |
| Czech | 52 | 70 | 6 |
| South - Italian | 37 | 72 | 7 |

**Table 2: Amount of defensin repeats, according number of patients and mean FEV1% value**

| **Repeat** | **Individuals** | **Mean FEV1%** |
|---|---|---|
| 3 | 3 | 38 |
| 4 | 14 | 56 |
| 5 | 32 | 68 |
| 6 | 34 | 77 |
| 7 | 19 | 71 |
| 8 | 20 | 76 |
| 9 | 3 | 84 |
| >=10 | 10 | 86 |

**Table 3: Division of the data set into three groups with the corresponding FEV1% values and the standard deviation**

| **Group** | **Repeat** | **Individuals** | **Mean FEV1%** | **SD** |
|---|---|---|---|---|
| 1 | 3 and 4 | 17 | 53 | 20 |
| 2 | 5 and 6 | 66 | 72 | 20 |
| 3 | >6 | 62 | 80 | 24 |

**Table 4: Statistical analyses of the different population with a student t-test.**

| **Population** | **Repeat** | **Individuals** | **Mean FEV1%** | **P-value** |
|---|---|---|---|---|
| Belgian | 4-5 | 18 | 63.26 | 0.013 |
| | >5 | 28 | 80.09 | |
| Czech | 3-5 | 24 | 63.63 | 0.048 |
| | >5 | 28 | 76.15 | |
| South-Italian | 4-6 | 12 | 60.26 | 0.044 |
| | >6 | 24 | 77.14 | |

## Claims

1. An assay kit for the determination of the copy number of a variable copy-number sequence in a genetic sample comprising a set of at least two control constructs, wherein each control construct is an isolated nucleic acid sequence comprising the same copy number of a reference sequence and a known but different number of copies of a repeated sequence.

2. An assay kit according to claim 1 wherein the reference sequence comprised in said control constructs corresponds to a reference sequence in the genetic sample and wherein the repeated sequence in the control constructs corresponds to a part of the said variable copy-number sequence.

3. An assay kit according to claim 2, comprising two sets of PCR primers wherein the first set of PCR primers allows the amplification of both a reference sequence in the genetic sample and the corresponding reference sequence in the control construct and wherein the second set of primers allows the amplification of a part of the variable copy-number sequence in the genetic sample and the corresponding repeated sequence in the control constructs.

4. An assay kit according to claim 1, comprising two sets of PCR primers wherein the first set of primers allows the amplification of a reference sequence in the genetic sample and wherein the second set of primers allows the amplification of a part of the variable copy-number sequence in the genetic sample and wherein neither the reference and repeated sequences in the control constructs are homologous with the reference sequence in the genetic sample or with the part of the variable copy-number sequence.

5. An assay kit according to claim 4, wherein at least one primer of the reference sequence comprises a 5' adapter sequence, said sequence corresponding to at least a part of the reference sequence comprised in the control constructs and wherein at least one primer of the variable copy-number sequence comprises a 5' adapter sequence, said sequence corresponding to at least a part of the repeated sequence comprised in the control constructs.

6. A method for the determination of the copy number of a variable copy-number sequence in a genetic sample using quantitative PCR and an assay kit according to any of the claims 1 to 5.

7. The method according to claim 6, wherein an assay kit according to claim 3 is used, said method comprising following steps:
(i) amplifying the reference sequence and the repeated sequence for each control construct using the said primers and if necessary isolating the PCR products from the non-reacted primers;
(ii) obtaining values reflecting the concentration of the PCR products of respectively the reference sequence and the repeated sequence for each control construct and normalising the values obtained for the repeated sequence against the values obtained for the reference sequence;
(iii) setting a standard curve indicating the relation between the copy number of the repeated sequence and the said normalised values;
(iv) amplifying the reference sequence and the part of the variable copy-number sequence in the genetic sample using the said primers and if necessary isolating the PCR products from the non-reacted primers;
(v) obtaining values reflecting the concentration of the PCR products of respectively the reference sequence and the part of the variable copy-number sequence in the genetic sample and normalising the value obtained for the part of the variable copy-number sequence against the value obtained for the reference sequence;
(vi) extrapolating the copy number of said variable copy-number sequence in the genetic sample using the normalised value obtained in step (v) and the standard curve obtained in step (iii).

8. The method according to claim 7, wherein the values reflecting the concentrations of the respective PCR products are obtained by using PCR primers comprising a label allowing the quantitative detection of the PCR products.

9. The method according to claim 7, wherein the values reflecting the concentrations of the respective PCR products are obtained after binding the PCR products to a solid surface and hybridising the bound PCR products with sequences specific to the respective PCR products.

10. The method according to claim 7, wherein the values reflecting the concentrations of the respective PCR products are obtained after hybridisation of the PCR products with sequences specific to the respective PCR products, said sequences being bound to a solid surface.

11. The method according to claims 9 or 10, wherein the solid surface is a membrane or a bead.

12. The method according to claim 6, wherein an assay kit according to claim 3 is used in a quenching based real time PCR system, said method comprising following steps:
(i) amplifying the reference sequence and the repeated sequence of each of the control constructs using primers, which are sequence specific for the reference sequence and the repeated sequence, respectively;
(ii) determining the cycle threshold (Ct-value) of the reference and the repeated sequence, respectively, for each control construct;
(iii) calculating the difference between the Ct-value of the reference sequence and the Ct-value obtained for the repeated sequence (Ct-ref - Ct-repeated) for each control construct;
(iv) determining a relationship between the number of copies of the repeated sequence, and the difference between Ct-ref - Ct-repeated;
(v) using this relationship to calculate the copy number of the variable copy-number sequence in a genetic sample comprising an unknown copy number after determination of the Ct-ref - Ct-variable for that sample, preferably using the same primers as used in step (i).

13. The method according to claim 6, wherein the assay kit according to claim 5 is used, said method comprising following steps:
(i) amplifying the reference sequence and a part of the variable copy-number sequence comprised in the genetic sample and if necessary isolating the PCR products from the non-reacted primers;
(ii) hybridising the PCR products with sequences complementary to the adapters of the primers of the reference and variable copy number sequence, respectively, and measuring the obtained hybridisation signals;
(iii) normalising the hybridisation signal obtained for the variable copy-number sequence against the signal obtained for the reference sequence;
(iv) hybridising the said control constructs with the same sequences used in step (ii) and measuring for each control construct the hybridisation signal for respectively the reference sequence and the repeated sequence;
(v) normalising for each control construct the signal obtained for the repeated sequence against the signal obtained for the reference sequence and using said normalised signals to set a standard curve indicating the relation between the copy number of the repeated sequence and the obtained normalised signals;
(vi) extrapolating the copy number of the variable copy-number sequence using the standard curve obtained in step (v) and the normalised signal obtained in step (iii).

14. A method for predicting the predisposition of an individual to develop a disease or for correlating the severity of a disease in an individual based on the assessment of the copy number of the variable copy-number sequence comprising the sequences encoding beta-defensins 2, 3, 4, 5 and 6 in a genetic sample isolated from said individual, wherein the copy number of said beta-defensin sequences is determined using an assay kit according to any one of claims 1 to 5.

15. The method according to claim 14, wherein the disease is a lung disease.

16. The method according to claim 15, wherein the disease is cystic fibrosis.

17. The method according to claim 15, wherein the disease is a chronic obstructive pulmonary disorder.

18. The method according to claim 17, wherein the chronic obstructive pulmonary disorder is emphysema.

19. The method according to claim 14, wherein the copy number of said beta-defensin sequences is determined using an assay kit according to any one of claims 1 to 3 and wherein the reference sequence in the control constructs corresponds to a part of the human beta-defensin 1 gene.

20. The method according to claim 14, wherein the copy number of said beta-defensin sequences is determined using an assay kit according to any one of claims 1 to 3 and wherein the repeated sequence in the control constructs corresponds to a part of the human beta-defensin 2 gene.

## Patentansprüche

1. Assay Kit zur Bestimmung der Kopienanzahl einer Sequenz mit variabler Kopien-Anzahl in einer genetischen Probe, die einen Satz von zumindest zwei Kontroll-Konstrukten umfasst, wobei jedes Kontroll-Konstrukt eine isolierte Nukleinsäuresequenz ist, die dieselbe Kopienanzahl einer Referenzsequenz und eine bekannte, jedoch unterschiedliche Anzahl von Kopien an einer wiederholten Sequenz umfasst.

2. Assay Kit nach Anspruch 1, wobei die Referenzsequenz, die in den Kontroll-Konstrukten vorhanden ist, eine Referenzsequenz in der genetischen Probe entspricht und wobei die wiederholte Sequenz in den Kontroll-Konstrukten einem Teil der Sequenz mit variabler Kopien-Anzahl entspricht.

3. Assay Kit nach Anspuch 2, der zwei Sätze von PCR-Primern umfasst, wobei der erste Satz von PCR-Primern die Amplifikation sowohl einer Referenzsequenz in der genetischen Probe als auch der entsprechenden Referenzsequenz im Kontroll-Konstrukt umfasst, und wobei der zweite Satz von Primern die Amplifikation eines Teils der Sequenz mit variabler Kopien-Anzahl und der entsprechenden wiederholten Sequenz in den Kontroll-Konstrukten ermöglicht.

4. Assay Kit nach Anspruch 1, der zwei Sätze von PCR-Primern umfasst, wobei der erste Satz von Primern die Amplifikation einer Referenzsequenz in der genetischen Probe ermöglicht, und wobei der zweite Satz von Primern die Amplifikation eines Teils der Sequenz mit variabler Kopien-Anzahl in der genetischen Probe ermöglicht und wobei weder die Referenz noch die wiederholten Sequenzen in den Kontroll-Konstrukten mit der Referenzsequenz in der genetischen Probe oder mit dem Teil der Sequenz mit variabler Kopien-Anzahl homolog sind.

5. Assay Kit nach Anspruch 4, wobei zumindest ein Primer der Referenzsequenz eine 5' Adapter-Sequenz umfasst, wobei die Sequenz, zumindest einem Teil der Referenzsequenz entspricht, die in den Kontroll-Konstrukten vorhanden ist, und wobei zumindest ein Primer der Sequenz mit variabler Kopien-Anzahl einer 5' Adapter-Sequenz umfasst, wobei die Sequenz zumindest einem Teil der wiederholten Sequenz entspricht, die in den Kontroll-Konstrukten vorhanden ist.

6. Verfahren zur Bestimmung der Kopien-Anzahl einer Sequenz mit variabler Kopien-Anzahl in einer genetischen Probe unter Verwendung einer quantitativen PCR und eines Assay Kits gemäß einem der Ansprüche 1 bis 5.

7. Verfahren nach Anspruch 6, wobei ein Assay Kit nach Anspruch 3 verwendet wird, und wobei das Verfahren die folgenden Schritten umfasst:
(i) Amplifizieren der Referenzsequenz und der wiederholten Sequenz für jedes Kontroll-Konstrukt unter Verwendung der Primer und, falls notwendig, isolieren der PCR-Produkte aus den nicht-umgesetzten Primern;
(ii) Gewinnen von Werten, die die Konzentration der PCR-Produkte jeweils der Referenzsequenz und der wiederholten Sequenz für jedes Kontroll-Konstrukt widerspiegeln und normalisieren der Werte, die für die wiederholte Sequenz erzielt wurden, gegen die Werte, die für die Referenzsequenz erzielt wurden;
(iii) Einstellen einer Standardkurve, die das Verhältnis zwischen der Kopienanzahl der wiederholten Sequenz und der normalisierten Werte anzeigt;
(iv) Amplifizieren der Referenzsequenz und des Teils der Sequenz mit variabler Kopien-Anzahl in der genetischen Probe unter Verwendung der Primer und, falls notwendig, isolieren der PCR-Produkte aus den nicht-umgesetzten Primern;
(v) Gewinnen der Werte, die die Konzentration der PCR-Produkte jeweils der Referenzsequenz und des Teils der Sequenz mit variabler Kopien-Anzahl in der genetischen Probe widerspiegelt und normalisieren des Wertes, der für den Teil der Sequenzen variabler Kopien-Anzahl erzielt wurde gegen den Wert, der für die Referenzsequenz erzielt wurde;
(vi) Extrapolieren der Kopien-Anzahl der Sequenz mit variabler Kopien-Anzahl in der genetischen Probe unter Verwendung des standardisierten Wertes, der in Schritt (v) erzielt wurde und der Standardkurve, die in Schritt (iii) erzielt wurde.

8. Verfahren nach Anspruch 7, wobei die Werte, die die Konzentrationen der jeweiligen PCR-Produkte widerspiegeln durch Verwendung von PCR-Primern gewonnen werden, die einen Marker umfassen, der den quantitativen Nachweis der PCR-Produkte ermöglicht.

9. Verfahren nach Anspruch 7, wobei die Werte, die die Konzentrationen der jeweiligen PCR-Produkte widerspiegeln, nach Binden der PCR-Produkte an eine feste Oberfläche und Hybridisieren der gebundenen PCR-Produkte mit Sequenzen gewonnen werden, die für die jeweiligen PCR-Produkte spezifisch sind.

10. Verfahren nach Anspruch 7, wobei die Werte, die die Konzentrationen der jeweiligen PCR-Produkte widerspiegeln nach Hybridisierung der PCR-Produkte mit Sequenzen gewonnen werden, die für die jeweiligen PCR-Produkte spezifisch sind, wobei die Sequenzen an eine feste Oberfläche gebunden sind.

11. Verfahren nach Anspruch 9 oder 10, wobei die feste Oberfläche eine Membrane oder eine Perle ist.

12. Verfahren nach Anspruch 6, wobei ein Assay Kit nach Anspruch 3 in einem löschungsbasiertem Echtzeit PCR-System verwendet wird, wobei das Verfahren die folgenden Schritte umfasst:
(i) Amplifizieren der Referenzsequenz und der wiederholten Sequenz jeder der Kontroll-Konstrukte unter der Verwendung von Primern, die jeweils für die Referenzsequenz und die wiederholte Sequenz spezifisch sind,
(ii) Bestimmen des Zyklusschwellenwerts (Ct-Wertes) der Referenz und der wiederholten Sequenz für jedes Kontroll-Konstrukt;
(iii) Berechnen des Unterschieds zwischen dem Ct-Wert der Referenzsequenz und dem Ct-Wert, der für die wiederholte Sequenz erzielt wurde (Ct-Ref - Ct-wiederholt) für jedes Kontroll-Konstrukt;
(iv) Bestimmen des Verhältnisses zwischen der Anzahl der Kopien der wiederholten Sequenz und des Unterschiedes zwischen Ct-Ref - Ct-wiederholt;
(v) Unter Verwendung dieses Verhältnisses berechnen der Kopien-Anzahl der Sequenz mit variabler Kopien-Anzahl in einer genetischen Probe, die eine unbekannte Kopien-Anzahl umfasst, nach Bestimmung der Ct-Ref - Ct-Variabel für diese Probe, vorzugsweise unter Verwendung der selben Primer wie sie in Schritt (i) verwendet wurden.

13. Verfahren nach Anspruch 6, wobei der Assay Kit nach Anspruch 5 verwendet wird, und wobei das Verfahren die folgenden Schritte umfasst:
(i) Amplifizierung der Referenzsequenz und eines Teils der Sequenz mit variabler Kopien-Anzahl, die in der genetischen Probe vorhanden ist, und falls notwendig, Isolieren der PCR-Produkte aus den nicht-reagierten Primern;
(ii) Hybridisieren der PCR-Produkte mit Sequenzen, die zu den Adaptern der Primer der Referenz und der Sequenz mit variabler Kopien-Anzahl komplementär sind, und Messen der gewonnenen Hybridisierungssignale;
(iii) Normalisieren des Hybridisierungssignals, das für die Sequenz mit variabler Kopien-Anzahl gewonnen wurde, gegen das Signal, das für die Referenzsequenz gewonnen wurde;
(iv) Hybridisieren der Kontroll-Konstrukte mit den selben Sequenzen, die in Schritt (ii) verwendet wurden und Messen für jedes Kontroll-Konstrukt des Hybridisierungssignals für jeweils die Referenzsequenz und die wiederholte Sequenz;
(v) Normalisieren für jedes Kontroll-Konstrukt des Signals, das für die wiederholte Sequenz erzielt wurde gegenüber dem Signal, das für die Referenzsequenz gewonnen wurde und Verwendung der normalisierten Signale zur Einstellung einer Standardkurve, die das Verhältnis zwischen der Kopien-Anzahl der wiederholten Sequenz und der erzielten normalisierten Signale angibt;
(vi) Extrapolieren der Kopien-Anzahl der Sequenz mit variabler Kopien-Anzahl unter Verwendung der in Schritt (v) erzielten Standardkurve und des normalisierten Signals, das in Schritt (iii) gewonnen wurde.

14. Verfahren zur Vorhersage der Prädisposition eines Individuums, eine Krankheit zu entwickeln oder zum Korrelieren des Schweregrades einer Erkrankung in einem Individuum auf Grundlage der Einschätzung der Kopien-Anzahl der Sequenz mit variabler Kopien-Anzahl, umfassend die Sequenzen, die die beta-Defensine 2, 3, 4, 5 und 6 kodieren, in einer genetischen Probe, die aus dem Individuum isoliert wurde, wobei die Kopien-Anzahl der beta-Defensin Sequenzen unter Verwendung eines Assay Kits gemäß einem der Ansprüche 1 bis 5 bestimmt wird.

15. Verfahren nach Anspruch 14, wobei die Krankheit eine Lungenkrankheit ist.

16. Verfahren nach Anspruch 15, wobei die Krankheit Zystische Fibrose ist.

17. Verfahren nach Anspruch 15, wobei die Krankheit eine chronisch obstruktive Lungenkrankheit ist.

18. Verfahren nach Anspruch 17, wobei die chronische obstruktive Lungenkrankheit ein Emphysem ist.

19. Verfahren nach Anspruch 14, wobei die Kopienanzahl der beta-Defensin Sequenzen unter Verwendung eines Assay Kits gemäß einem der Ansprüche 1 bis 3 bestimmt wird und wobei die Referenzsequenz in den Kontroll-Konstrukten einen Teil des humanen beta-Defensinl Gens entspricht.

20. Verfahren nach Anspruch 14, wobei die Kopienanzahl der beta-Defensin Sequenzen unter Verwendung eines Assay Kits gemäß einem der Ansprüche 1 bis 3 bestimmt wird und wobei die wiederholte Sequenz in den Kontroll-Konstrukten einen Teil des humanen beta-Defensin2 Gens entspricht.

## Revendications

1. Kit de dosage destiné à la détermination du nombre de copies d'une séquence à nombre de copies variable dans un échantillon génétique comprenant un jeu d'au moins deux constructions de contrôle, dans lequel chaque construction de contrôle est une séquence d'acide nucléique isolée comprenant le même nombre de copies d'une séquence référence et un nombre de copies connu mais différent d'une séquence répétée.

2. Kit de dosage selon la revendication 1, dans lequel la séquence référence comprise dans lesdites constructions de contrôle correspond à une séquence référence dans l'échantillon génétique et dans lequel la séquence répétée dans les constructions de contrôle correspond à une partie de ladite séquence à nombre de copies variable.

3. Kit de dosage selon la revendication 2, comprenant deux jeux d'amorces PCR, dans lequel le premier jeu d'amorces PCR permet l'amplification à la fois d'une séquence référence dans l'échantillon génétique et de la séquence référence correspondante dans la construction de contrôle et dans lequel le second jeu d'amorces permet l'amplification d'une partie de la séquence à nombre de copies variable dans l'échantillon génétique et la séquence répétée correspondante dans les constructions de contrôle.

4. Kit de dosage selon la revendication 1 comprenant deux jeux d'amorces PCR, dans lequel le premier jeu d'amorces permet l'amplification d'une séquence référence dans l'échantillon génétique et dans lequel le second jeu d'amorces permet l'amplification d'une partie de la séquence à nombre de copies variable dans l'échantillon génétique et dans lequel ni la référence et ni les séquences répétées dans les constructions de contrôle ne sont homologues avec la séquence référence dans l'échantillon génétique ou avec la partie de la séquence à nombre de copies variable.

5. Kit de dosage selon la revendication 4, dans lequel au moins une amorce de la séquence référence comprend une séquence de l'adaptateur 5', ladite séquence correspondant à au moins une partie de la séquence référence comprise dans les constructions de contrôle et dans lequel au moins une amorce de la séquence à nombre de copies variable comprend une séquence de l'adaptateur 5', ladite séquence correspondant à au moins une partie de la séquence répétée comprise dans les constructions de contrôle.

6. Procédé de détermination du nombre de copies d'une séquence à nombre de copies variable dans un échantillon génétique utilisant la PCR quantitative et un kit de dosage selon l'une quelconque des revendications 1 à 5.

7. Procédé selon la revendication 6, dans lequel un kit de dosage selon la revendication 3 est utilisé, ledit procédé comprenant les étapes suivantes consistant à :
(i) amplifier la séquence référence et la séquence répétée pour chaque construction de contrôle en utilisant lesdites amorces et si nécessaire isoler les produits PCR des amorces n'ayant pas réagi ;
(ii) obtenir les valeurs qui reflètent la concentration des produits PCR, respectivement, de la séquence référence et de la séquence répétée pour chaque construction de contrôle et normaliser les valeurs obtenues pour la séquence répétée par rapport aux valeurs obtenues pour la séquence référence ;
(iii) établir une courbe standard indiquant la relation entre le nombre de copies de la séquence répétée et lesdites valeurs normalisées ;
(iv) amplifier la séquence référence et la partie de la séquence à nombre de copies variable dans l'échantillon génétique en utilisant lesdites amorces et si nécessaire isoler les produits PCR des amorces n'ayant pas réagi ;
(v) obtenir les valeurs qui reflètent la concentration des produits PCR, respectivement, de la séquence référence et de la partie de la séquence à nombre de copies variable dans l'échantillon génétique et normaliser la valeur obtenue pour la partie de la séquence à nombre de copies variable par rapport à la valeur obtenue pour la séquence référence ;
(vi) extrapoler le nombre de copies de ladite séquence à nombre de copies variable dans l'échantillon génétique en utilisant la valeur normalisée obtenue dans l'étape (v) et la courbe standard obtenue dans l'étape (iii).

8. Procédé selon la revendication 7, dans lequel les valeurs, qui reflètent les concentrations des produits PCR respectifs, sont obtenues en utilisant des amorces PCR comprenant un marqueur permettant la détection quantitative des produits PCR.

9. Procédé selon la revendication 7, dans lequel les valeurs, qui reflètent les concentrations des produits PCR respectifs, sont obtenues après liaison des produits PCR à une surface solide et hybridation des produits PCR liés ayant des séquences spécifiques aux produits PCR respectifs.

10. Procédé selon la revendication 7, dans lequel les valeurs qui reflètent les concentrations des produits PCR respectifs sont obtenues après hybridation des produits PCR ayant des séquences spécifiques aux produits PCR respectifs, lesdites séquences étant liées à une surface solide.

11. Procédé selon les revendications 9 ou 10, dans lequel la surface solide est une membrane ou une perle.

12. Procédé selon la revendication 6, dans lequel un kit de dosage selon la revendication 3 est utilisé dans un système PCR en temps réel basé sur le recuit, ledit procédé comprenant les étapes suivantes consistant à :
(i) amplifier la séquence référence et la séquence répétée de chacune des constructions de contrôle en utilisant des amorces qui sont spécifiques à la séquence, respectivement, pour la séquence référence et la séquence répétée ;
(ii) déterminer le cycle seuil (valeur de Ct), respectivement, de la référence et de la séquence répétée pour chaque construction de contrôle ;
(iii) calculer la différence entre la valeur de Ct de la séquence référence et la valeur de Ct obtenue pour la séquence répétée (Ct-réf - Ct-répétée) pour chaque construction de contrôle ;
(iv) déterminer une relation entre le nombre de copies de la séquence répétée, et la différence Ct-réf - Ct-répétée ;
(v) utiliser cette relation pour calculer le nombre de copies de la séquence à nombre de copies variable dans un échantillon génétique comprenant un nombre de copies inconnu après détermination de la différence Ct-réf - Ct-variable pour cet échantillon, de préférence en utilisant les mêmes amorces que celles utilisées dans l'étape (i).

13. Procédé selon la revendication 6, dans lequel le kit de dosage selon la revendication 5 est utilisé, ledit procédé comprenant les étapes suivantes consistant à :
(i) amplifier la séquence référence et une partie de la séquence à nombre de copies variable comprise dans l'échantillon génétique et si nécessaire isoler les produits PCR des amorces n'ayant pas réagi ;
(ii) hybrider les produits PCR ayant des séquences complémentaires aux adaptateurs des amorces, respectivement, de la référence et de la séquence à nombre de copies variable et mesurer les signaux d'hybridation obtenus ;
(iii) normaliser le signal d'hybridation obtenu pour la séquence à nombre de copies variable par rapport au signal obtenu pour la séquence référence ;
(iv) hybrider lesdites constructions de contrôle avec les mêmes séquences utilisées dans l'étape (ii) et mesurer pour chaque construction de contrôle le signal d'hybridation pour respectivement la séquence référence et la séquence répétée ;
(v) normaliser pour chaque construction de contrôle le signal obtenu pour la séquence répétée par rapport au signal obtenu pour la séquence référence et utiliser lesdits signaux normalisés pour établir une courbe standard indiquant la relation entre le nombre de copies de la séquence répétée et les signaux normalisés obtenus ;
(vi) extrapoler le nombre de copies de la séquence à nombre de copies variable en utilisant la courbe standard obtenue dans l'étape (v) et le signal normalisé obtenu dans l'étape (iii).

14. Procédé de prédiction de la prédisposition d'un individu à développer une maladie ou de corrélation de la sévérité d'une maladie chez un individu, basé sur l'estimation du nombre de copies de la séquence à nombre de copies variable comprenant les séquences encodant les β-défensines 2, 3, 4, 5 et 6 dans un échantillon génétique isolé dudit individu, dans lequel le nombre de copies desdites séquences de β-défensine est déterminé en utilisant un kit de dosage selon l'une quelconque des revendications 1 à 5.

15. Procédé selon la revendication 14, dans lequel la maladie est une maladie des poumons.

16. Procédé selon la revendication 15, dans lequel la maladie est une fibrose cystique.

17. Procédé selon la revendication 15, dans lequel la maladie est un trouble pulmonaire obstructif chronique.

18. Procédé selon la revendication 17, dans lequel le trouble pulmonaire obstructif chronique est un emphysème.

19. Procédé selon la revendication 14, dans lequel le nombre de copies desdites séquences β-défensines est déterminé en utilisant un kit de dosage selon l'une quelconque des revendications 1 à 3 et dans lequel la séquence référence dans les constructions de contrôle correspond à une partie du gène β-défensine 1 humain.

20. Procédé selon la revendication 14, dans lequel le nombre de copies desdites séquences β-défensines est déterminé en utilisant un kit de dosage selon l'une quelconque des revendications 1 à 3 et dans lequel la séquence répétée dans les constructions de contrôle correspond à une partie du gène β-défensine 2 humain.
